# EUROPÄISCHE PATENTANMELDUNG

(11) **EP 3 834 874 A1**
(43) Veröffentlichungstag der Anmeldung: **16.06.2021**
(21) Anmeldenummer: 19215220.5
(22) Anmeldetag: 11.12.2019
(51) Int. Cl.: A61M 25/00

(54) **KATHETER MIT SCORINGELEMENT**

(71) Anmelder: BIOTRONIK AG, 8180 Bülach (CH)
(72) Erfinder: Nollert, Georg, 82064 Strasslach (DE)
(74) Vertreter: Randoll, Sören

(57) **Zusammenfassung**

Katheter zum Aufweiten und/ oder Zerstören einer Stenose in einem Gefäß eines Patienten, umfassend einen Katheterschaft (20) mit einem Hohlraum (24) und in dem Hohlraum (24) zumindest ein erstes, mit einer den Hohlraum (24) begrenzenden Wandung (25) fest verbundenes erstes Wirkelement (26) zur Ausbildung des Wirkprinzips der geneigten Ebene (50) zwecks Vergrößerung eines Abstandes (A) senkrecht zu einer Längsachse (21) des Katheterschaftes (20) und dadurch bedingter radialer Aufweitung des Katheterschaftes (20). Erfindungsgemäß ist vorgesehen, dass der Katheter (1) an der Außenseite des Katheterschaftes (20) angeordnet wenigstens ein radial vorstehendes Druckelement (30) zur Ausübung einer Druckkraft (31) auf eine Stenose in einem Gefäß aufweist.

## Beschreibung

Die Erfindung betrifft einen expandierbaren Katheter mit einem Scoringelement.

Zur Präparation von fibrösen und kalzifizierten Stenosen werden zur Prädilatation häufig Ballon-Katheter in Form von sogenannten Scoring- bzw. Cutting-Ballonen verwendet. Ein derartiger als Scoring-Ballonkatheter weist einen Ballon auf, auf dessen nach außen gewandter Oberfläche zumindest ein längserstrecktes Kontaktelement festgelegt ist, das auch als Scoringelement bezeichnet wird.

Weiterhin sind aus dem Stand der Technik Scoringelemente bekannt, die durch ein Drahtgeflecht gebildet sind oder durch einzelne mit der Balloberfläche verklebte Elemente. Diese Ballone schneiden oder ritzen in die Intima und Media der Arterie und brechen so Verkalkungen auf oder durchschneiden Vernarbungen (Fibrosierungen) der Gefäßverengungen.

Scoring-Ballone mit umlaufenden Schneiden haben sich hier als weniger verletzend im Vergleich zu Cutting-Ballonen mit geraden Schneiden gezeigt. Diese Ballone haben einen definierten Durchmesser und Länge, so dass viele dieser Ballone auf Lager gehalten werden müssen, um unterschiedliche Stenosen behandeln zu können.

Des Weiteren kann es bei der Verwendung von Ballonen, wenn sich diese an eine Gefäßverengung anlegen, zu Einschnürungen bzw. radialen Verengungen der Ballone kommen, auf Grund einer geringeren Härte der Außenseite der Ballone gegenüber der Stenose. Dies führt zu einer Vergrößerung der Anlagefläche des Ballons an der Stenose und somit zu einer Verringerung des aufgebrachten Drucks auf die Stenose und läuft somit der beabsichtigten Behandlungswirkung zuwider.

Zudem bedeutet dies, dass der Ballon bei starken Verhärtungen an diesen Stellen nachgibt und dass der harte Teil der Gefäßverengung nicht so aufgeweitet wird wie der restliche Teil der Gefäßverengung.

Hiervon ausgehend liegt der vorliegenden Erfindung daher die Aufgabe zugrunde, einen Katheter sowie ein Katheter-System zur Verfügung zu stellen, mit denen in kostengünstiger sowie zuverlässiger Weise Stenosen unterschiedlicher geometrischer Ausbildung und Länge behandelt werden können.

Diese Aufgabe wird durch den erfindungsgemäßen Katheter nach Anspruch 1 sowie durch das erfindungsgemäße Katheter-System nach Anspruch 15 erfüllt. Vorteilhafte Ausgestaltungen des erfindungsgemäßen Katheters sind in den Unteransprüchen 2-14 angegeben.

Der erfindungsgemäße Katheter und seine vorteilhaften Ausgestaltungen werden nachfolgend beschrieben.

Gemäß Anspruch 1 wird ein Katheter zum Aufweiten einer Stenose eines Gefäßes eines Patienten offenbart, der einen Katheterschaft mit einem Hohlraum und in dem Hohlraum zumindest ein erstes, mit einer den Hohlraum begrenzenden Wandung fest verbundenes erstes Wirkelement zur Ausbildung des Wirkprinzips der geneigten Ebene zwecks Vergrößerung eines Abstandes senkrecht zu einer Längsachse des Katheterschaftes und dadurch bedingter radialer Aufweitung des Katheterschaftes aufweist. Der Katheter umfasst des Weiteren an der Außenseite des Katheterschaftes angeordnet wenigstens ein radial vorstehendes Druckelement zur Ausübung einer Druckkraft auf eine Stenose in einem Gefäß.

Die Begriffe "radial" und "axial" beziehen sich auf die Längsachse des Katheterschaftes. Der auch als Lumen bezeichnete Hohlraum ist im Wesentlichen an einem distalen Endabschnitt des Katheters im Inneren des Katheterschafts koaxial zur Außenseite ausgebildet. Insbesondere kann der Hohlraum axial einseitig offen ausgestaltet sein.

Bei Ausführung einer Bewegung eines Körpers auf der geneigten Ebene vergrößert dieser Körper seinen Abstand in Bezug zur Basis der geneigten Ebene. Diese Abstandsvergrößerung wird erfindungsgemäß zur radialen Aufweitung des Katheterschaftes genutzt. Dadurch kann das von der Außenseite des Katheterschaftes nach radial außen vorstehende Druckelement an eine Stenose bzw. Läsion gedrückt werden und diese aufweiten bzw. zerstören.

Entsprechend weist der erfindungsgemäße Katheter den Vorteil auf, dass durch eine einfache, insbesondere manuelle Betätigung des Wirkprinzips der geneigten Ebene eine radiale Aufweitung des Schaftes und entsprechend effizient und fein einstellbar eine Kraft in radialer Richtung auf eine Wand eines Gefäßes bzw. auf eine dort befindliche Stenose aufgebracht werden kann, um die Stenose mechanisch aufzuweiten bzw. zu zerstören.
Das Druckelement kann auch als Scoring- Element bezeichnet werden.
Der erfindungsgemäße Katheter ist entsprechend als Scoring-Katheter für ein sogenanntes Scoring zwecks Dilatation eines Gefäßes ausgestaltet.

Das erste Wirkelement kann dabei insbesondere fest an der den Hohlraum begrenzenden Wandung angeordnet sein oder ein integraler Bestandteil dieser Wandung sein.

Anstatt eines Ballons mit Schneiden beruht die Erfindung auf einem expandierbaren sogenannten Sheath mit Schneiden als Druckelemente. Durch das Vorbringen von auch als Dilatatoren genannten Vorschubelementen unterschiedlichen Durchmessers kann das Sheath verschiedene Durchmesser annehmen. Je nach Tiefe des Vorbingens des Dilatators ist auch die Länge des radial expandierenden Längenbereichs variabel.

Gemäß einer Ausführungsform der Erfindung ist vorgesehen, dass der Katheter des Weiteren ein Vorschubelement zur Einführung und/oder Bewegung in dem Hohlraum entlang der Längsachse des Katheterschaftes umfasst, wobei das Vorschubelement ein zweites Wirkelement zur Ausbildung des Wirkprinzips der geneigten Ebene aufweist, welches an dem ersten Wirkelement anliegt oder anlegbar ist, so dass bei Relativbewegung zwischen den beiden Wirkelementen eine Keilwirkung realisierbar ist, mit der eine radiale Aufweitung des Katheterschaftes erzeugbar ist.

Der Katheter bildet zu diesem Zweck an den Hohlraum angrenzend einen Kanal aus, durch welchen das Vorschubelement zu einem proximalen Endbereich des Katheters führt, zwecks manueller Betätigung des Vorschubelements am proximalen Ende.
Die Anlage des zweiten Wirkelements am ersten Wirkelement erfolgt nach Ausführung einer Vorschubbewegung des Vorschubelements.

Das Vorschubelement kann an seinem proximalen Endbereich wenigstens eine Markierung, insbesondere mehrere entlang der Längserstreckungsrichtung des Vorschubelements angeordnete Markierungen aufweisen, anhand derer eine den erfindungsgemäßen Katheter bedienende Person Informationen hinsichtlich der EinschubTiefe des Vorschubelements in den Katheterschaft und somit hinsichtlich der Länge der radialen Aufweitung erhält.
Der erfindungsgemäße Katheter weist den Vorteil auf, dass bei ausreichender Elastizität der den Hohlraum begrenzenden Wandung bzw. des Materials des Katheterschaftes Vorschubelemente unterschiedlicher radialer Weite bzw. unterschiedlicher Durchmesser in den Hohlraum eingeschoben werden können, die in entsprechender Weise unterschiedliche radiale Aufweitungen bewirken bzw. über die Keilwirkung in Abhängigkeit der radialen Verformbarkeit der Außenseite des Katheterschafts unterschiedliche radiale Kräfte realisieren.

In einer Ausführungsform ist vorgesehen, dass eines der beiden Wirkelemente eine Anlagefläche und das jeweils andere Wirkelement eine geneigte Fläche aufweist, so dass bei Anlage der geneigten Fläche an der Anlagefläche und Ausführung einer Vorschubbewegung des Vorschubelements das erste Wirkelement nach radial außen bewegt wird.

Diese Bewegung nach radial außen wird durch das Prinzip der geneigten Ebene bzw. durch die Keilwirkung entgegen einer gegebenenfalls vorhandenen elastischen Rückstellkraft des Katheterschaftes realisiert.
Eine Neigung ist dabei in Bezug zur Längsachse des Katheterschaftes definiert.
Insbesondere kann vorgesehen sein, dass das erste Wirkelement am Katheterschaft eine Anlagefläche aufweist und das zweite Wirkelement des Vorschubelements die geneigte Fläche aufweist. Allerdings ist auch eine umgekehrte Konfiguration der Wirkelemente nicht von der Erfindung ausgeschlossen. Zudem kann auch die Anlagefläche selbst geneigt sein bzw. ebenfalls eine geneigte Ebene ausbilden, die die Keilwirkung verstärkt.

Eine vorteilhafte Ausführungsform sieht vor, dass sich in axialer Richtung an die jeweilige Fläche jeweils ein Absatz anschließt, so dass nach keilwirkungsbedingter Aufweitung des Katheterschaftes und unter Ausführung einer weiteren Vorschubbewegung die Absätze radial aneinander zur Anlage bringbar sind.

Der jeweilige Absatz erstreckt sich dabei so weit radial wie der radial am weitesten entfernte Punkt der jeweiligen Fläche. Das bedeutet, dass sich der an die Anlagefläche anschließende Absatz so weit nach radial innen erstreckt wie die maximale radiale Erstreckung der Anlagefläche selbst, wenn diese am ersten Wirkelement und demzufolge am Katheterschaft angeordnet bzw. ausgebildet ist.

Der sich an die geneigte Fläche anschließende Absatz erstreckt sich so weit nach radial außen wie die maximale radiale Erstreckung der geneigten Fläche selbst, wenn die geneigte Fläche am zweiten Wirkelement und demzufolge am Vorschubelement angeordnet bzw. ausgebildet ist.

Die Länge des Einschubbereichs der Absätze bestimmt dabei die Länge der radialen Aufweitung bzw. Expansion des Katheterschaftes und somit die Länge, über die erfindungsgemäß Druckkraft auf die Gefäßwandung aufgebracht wird.

Mittels der Absätze wird des Weiteren sichergestellt, dass zumindest über die Länge der Absätze keine radiale Einschnürung der Außenseite des Katheterschaftes auftreten kann, wie sie gegebenenfalls bei Ballon-Kathetern auftritt.

Des weiteren kann sich an den zur radialen Aufweitung vorgesehenen Hohlraum ein sich in den distalen Endbereich des Katheters erstreckendes Lumen anschließen, welches zur Aufnahme eines Führungselements, wie zu Beispiel eines Führungsdrahtes, zwecks Einführung des Katheters in ein Gefäß eines Patienten dient.

Dabei kann im Übergangsbereich zwischen dem Hohlraum und diesem Lumen wenigstens eine nach radial innen vorstehende Schulter angeordnet sein, um eine mechanische Blockadewirkung einer Vorschubbewegung des Vorschubelements zu realisieren. Das distale Ende des erfindungsgemäßen Katheters ist vorzugsweise spitz bzw. konisch ausgeführt, um die Einführung in ein Gefäß zu erleichtern.

Vorteilhafterweise sind das erste Wirkelement sowie das zweite Wirkelement in einer Ebene senkrecht zur Längsachse vollständig um diese Längsachse herum ausgebildet.

Insbesondere ist vorgesehen, dass sowohl das erste Wirkelement als auch das zweite Wirkelement rotationssymmetrisch zur Längsachse diese umgebend ausgebildet sind.
So kann insbesondere das erste Wirkelement die Form eines Hohlkegelstumpfes aufweisen und das zweite Wirkelement zumindest abschnittsweise die Form eines Kegels aufweisen, der bei Bewegung in den Hohlkegelstumpf diesen radial aufweitet und derart eine radiale Aufweitung des Katheterschaftes insgesamt erreicht. Entsprechend ist in dieser Ausführungsform die geneigte Fläche die Mantelfläche eines Kegels oder Kegelstumpfes und die Anlagefläche die Mantelfläche eines Hohlkegelstumpfes.

Dabei soll auch eine Ausführung nicht von der Erfindung ausgeschlossen sein, bei der wenigstens eines der beiden Wirkelemente lediglich als auf einer Umfangsbahn um die Längsachse beabstandete Segmente ausgeführt ist, um eine radiale Aufweitung des Katheterschaftes zu erleichtern.

Zwecks Erleichterung einer Aufweitung kann geneigte Fläche und/oder die Anlagefläche in einer Ebene, in der die Längsachse liegt, einen gewölbten Verlauf aufweisen. Das bedeutet, dass eine jeweilige, das Keilprinzip realisierende Fläche konkav oder konvex gewölbt ausgeführt sein kann. Insbesondere kann die Anlagefläche des ersten Wirkelements eine konvexe Wölbung aufweisen, die es einem kegeligen zweiten Druckelement erleichtert, unter Selbstzentrierung in das erste Wirkelement eingeschoben zu werden und dabei das erste Wirkelement und damit den Katheterschaft zu expandieren.

In einer vorteilhaften Ausführungsform ist vorgesehen, dass der Katheterschaft eine umfangsseitig geschlossene Außenwandung aufweist, die den Hohlraum begrenzt, wobei die Außenwandung radial aufweitbar ist. Diese Ausgestaltung ist insbesondere in dem Längenabschnitt des Katheterschaftes realisiert, in dem der radial aufweitbare Hohlraum positioniert ist. Dies ist insbesondere ermöglicht durch die Verwendung eines elastisch dehnbaren Materials für die Außenwandung.

Das Vorschubelement kann eine biegbare Stange, insbesondere eine Schubstange, sein, zur axialen Verlagerung des zweiten Wirkelements.

Zur manuellen Betätigung des Vorschubelements ist insbesondere vorgesehen, dass diese Schubstange an dem proximalen Ende des Katheterschaftes aus diesem herausragt.

Das Druckelement kann eine längliche, entlang seiner Längserstreckung den Umfang des Katheterschaftes umschließende Ausgestaltung aufweisen.

Dabei kann das Druckelement helixförmig an der Außenseite des Katheterschaftes angeordnet sein.

Diese Ausführungsform hat den Vorteil, dass bei Ausführung einer Drehbewegung des Katheterschaftes zusätzlich zu einer radialen Komponente einer ausgeübten Druckkraft auch noch eine axiale Komponente auf eine Stenose ausübbar ist, sodass diese einer mehrachsigen Belastung ausgesetzt ist und dadurch effizienter und/ oder gründlicher beseitigbar ist.

Insbesondere kann das Druckelement in der Form einer Doppelhelix oder Dreifachhelix an der Außenseite des Katheterschaftes angeordnet sein.

Das Druckelement kann an seinem radial äußeren Bereich eine geringere Breite aufweisen als im Bereich seiner mechanischen Fixierung an der Außenseite des Katheterschaftes.

Dies hat den Vorteil der Ausübung einer konzentrierten, hohen Druckkraft auf eine Stenose zwecks deren Aufweitung und/ oder Aufspaltung bzw. Zertrümmerung.

In einer Ausführungsform des Katheters ist vorgesehen, dass das Druckelement distal anschließend an die Helixform einen Ring umfasst, der in axialer Richtung/in Umfangsrichtung an der Außenseite des Katheterschaftes fixiert ist. Durch einen solchen Ring kann verhindert werden, dass das Vorschubelement durch den Katheter über das Ende hinaus geschoben wird.

Das Material des Rings ist vorzugsweise eine Nickel-Titan-Legierung, die eine relativ große radiale Aufweitung ermöglicht und eine somit eine gewisse Flexilibilät. Insbesondere kann als Material des Rings eine Formgedächtnislegierung genutzt werden, wie zum Beispiel Nitinol.

Der Ring ist aber nicht zwangsweise axial an dem Druckelement zu fixieren, sondern es reicht aus, wenn der Ring sowie das Druckelement derart positioniert sind, dass bei einer Axial-Relativbewegung zwischen diesen beiden Bauteilen eine Druckkraft zwischen ihnen übertragbar ist.

Insbesondere ist ein derartiger Ring angeordnet, um in axialer Richtung die Position des Druckelements, insbesondere bei dessen Helix-förmiger Ausgestaltung, zu sichern.
Dabei ist ein derartiger Ring vorteilhafterweise außerhalb des axialen Längenabschnitts des Katheterschaftes angeordnet, welcher bei Einschub des Vorschubelements die radiale Aufweitung erfährt.

Das Material des Katheterschaftes ist vorzugsweise aus geeigneten Polymeren, auch in Form eines Komposits aus mehr als einem Polymer oder einem Komposit aus einem oder mehreren Polymeren und einer Metallkomponente gefertigt. Beispiele für Polymere sind Polypropylen, Polyethylen, Polyethylenterephthalat, Polyamid, Polyimid, Polyamid-Copolymere, Polyetherblockamid, Silikon oder silikon-basierte Materialien.
Das Material des Druckelements ist beispielsweise aus Nitinol, Edelstahl, Polycarbonat.

Die verwendeten Materialien gewährleisten einerseits eine zuverlässige radiale Kraftübertragung zwecks Ausübung eines Drucks auf eine Stenose bei gleichzeitiger relativ geringer Biegesteifigkeit zur Anpassung des Katheters bzw. Katheterschaftes an die Windungen des Gefäßsystems.

Ein weiterer Bestandteil des erfindungsgemäßen Katheters kann eine äußere Umhüllung sein, auch äußeres Sheath genannt, welches mittels eines Führungselements, wie zu Beispiel einem Führungsdraht, in das zu behandelnde Gefäß eingeschoben werden kann. Dieses Führungselement dient dann ebenfalls zur Führung und Positionierung des eigentlichen Katheterschaftes.

Ferner wird ein Katheter- System offenbart, welches wenigstens einen erfindungsgemäßen Katheter sowie mehrere Vorschubelemente unterschiedlicher radialer Weite umfasst.

Das bedeutet, dass das Katheter-System zumindest einen erfindungsgemäß ausgestalteten Katheterschaft aufweist und einen Satz unterschiedlicher Vorschubelemente, deren jeweilige zweite Wirkelemente unterschiedlich große radiale Erstreckungen aufweisen, um in Zusammenwirkung mit dem Katheterschaft bzw. dem dortigen ersten Wirkelement unterschiedlich große radiale Expansionen zu erreichen.
Der Vorteil dieses erfindungsgemäßen Katheter-Systems besteht insbesondere darin, dass bei Vorhalten lediglich eines Katheterschaftes und Auswahl des geeigneten Vorschubelements Stenosen in einem unterschiedlichen Durchmesser-Bereich und auch unterschiedlicher Länge optimal und zuverlässig behandelt werden können, ohne den Katheterschaft wechseln zu müssen.
Der Vorteil des erfindungsgemäßen Katheters bzw. Katheter-Systems liegt insbesondere in der Reduktion der benötigten Geräte, da lediglich nur ein Katheterschaft vorgehalten werden muss und unterschiedliche, konstruktiv einfach ausgeführte Vorschubelemente unterschiedlicher radialer Größe, um Stenosen unterschiedlicher lichter Weite bzw. unterschiedlicher Längen optimal behandeln zu können. Mit einem derartigen System ist es weiterhin möglich, schrittweise eine Aufweitung bzw. Behandlung von Stenosen auszuführen, indem sequenziell entsprechend der Zunahme der radialen Weite der unterschiedlichen Vorschubelemente bzw. Dilatatoren diese im selben Katheterschaft genutzt werden.

Des Weiteren sind unbeabsichtigte radiale Einschnürungen, wie sie bei Ballonen häufiger auftreten, beim erfindungsgemäßen Katheter nicht zu erwarten, da dessen Außenseite aus einem deutlich härteren bzw. festeren Material gefertigt sein kann als ein expandierter Ballon und zudem die feste mechanische Verbindung durch das Prinzip der geneigten Ebene im Inneren des Katheterschaftes für eine definierte mechanische Abstützung sorgt, so dass keine unbeabsichtigte radiale Verengung des Katheters auftreten kann.

Weitere Merkmale und Vorteile der Erfindung sollen bei der Figurenbeschreibung eines Ausführungsbeispiels der Erfindung anhand der Figur erläutert werden. Es zeigt:

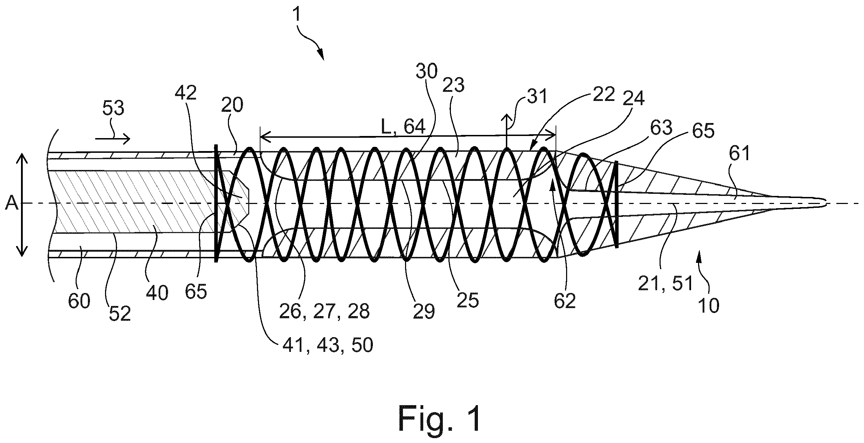

Fig. 1 eine Schnittansicht einer Ausführungsform eines erfindungsgemäßen Katheters.

Die Figur 1 zeigt eine Ausführungsform eines erfindungsgemäßen Katheters 1, der zum Aufweiten einer Stenose in einem Gefäß eines Patienten dient. Der Katheter 1 weist hierzu einen flexiblen, entlang einer Längsachse 21 erstreckten Katheterschaft 20 auf, der sich von einem hier nicht dargestellten proximalen Endabschnitt hin zu einem distalen Endabschnitt 10 erstreckt. Der Katheterschaft 20 weist an der Außenseite 22 einer Außenwandung 23, die im Wesentlichen zylinderförmig ausgebildet ist, ein hier beispielhaft in Form einer Doppelhelix ausgebildetes Druckelement 30 aus.

Dieses Druckelement 30 kann mit der Außenwandung 23 fest verbunden sein. Das Druckelement 30 ragt radial aus der Außenwandung 23 hervor. Es liegt an seinen beiden axialen Endbereichen an jeweils einem Ring 65 an, der jedoch selbst in Umfangsrichtung nicht fixiert sein muss.
Das Druckelement 30 erstreckt sich dabei in Längsrichtung des Katheterschaftes 20 über einen Bereich hinweg, in dem die Außenwandung 23 des Katheterschaftes 20 einen inneren Hohlraum 24 begrenzend einen ersten Absatz 29 ausbildet. Dieser erste Absatz 29 begrenzt somit mit einer inneren Wandung 25 den Hohlraum 24.

An einem dem distalen Endabschnitt 10 abgewandten Endbereich des ersten Absatzes 29 hat dieser ein erstes Wirkelement 26, welches eine Anlagefläche 27 mit einer konvexen Wölbung 28 aufweist.
In einem Kanal 60 im Katheterschaft 20 ist entlang der Richtung der Längsachse 21 beweglich ein Vorschubelement 40 in Form einer Druckstange angeordnet. An seinem den distalen Endabschnitt 10 zugewandten Endbereich weist das Vorschubelement 40 ein zweites Wirkelement 41 auf, welches in Form eines Kegelstumpfes 42 eine geneigte Fläche 43 definiert, die an dem ersten Wirkelement 26 bzw. dessen Anlagefläche 27 zur Anlage gebracht werden kann.
Das erste Wirkelement und das zweite Wirkelement bilden dann zusammen das Wirkprinzip der geneigten Ebene 50 aus, die es ermöglicht, bei einer Vorschubbewegung 53 entlang der Längsachse 21 die Vergrößerung eines Abstandes A in Bezug zur Basis 51 der geneigten Ebene 50 mit geringem Kraftaufwand zu realisieren.
Entsprechend fährt bei weiterer Vorschubbewegung 53 des Druckelements 30 der Kegelstumpf 42 des Vorschubelements 40 in das erste Wirkelement 26, welches im Wesentlichen als ein Hohlkegel ausgebildet ist, einfährt und derart das erste Wirkelement 26 radial aufweitet. Aufgrund der festen Verbindung des ersten Wirkelements 26 mit der Außenwandung 23 des Katheterschaftes 20 kommt es insgesamt zu einer Vergrößerung des Abstandes A und somit zu einer radialen Aufweitung des Katheterschaftes 20.

Entsprechend lässt sich eine Druckkraft 31 in radialer Richtung von der Außenseite 22 des Katheterschaftes 20 und von dem dort angeordneten Druckelement 30 auf eine den Katheterschaft 20 umgebende Stenose bzw. Gefäßwand ausüben.
Wenn das Vorschubelement 40 derart weit verschoben wurde, dass die beiden Wirkelemente 26,41 aneinander vorbei bewegt wurden, liegen der erste Absatz 29 vom Katheterschaft 20 sowie der zweite Abs. 52 vom Vorschubelement 40 in radialer Richtung aneinander an.
Dies führt zu einer sehr hohen Druckfestigkeit des Katheters 1 in dem Überlagerungsbereich.
Je nach Tiefe des Einschubs des Vorschubelements 40 in den Katheterschaft 20 lässt sich die Länge des axialen Längenabschnitts der radialen Aufweitung 64 bestimmen. Es ist ersichtlich, dass die Länge des axialen Längenabschnitts der radialen Aufweitung 64 nicht größer sein kann als die Länge L des ersten Absatzes, da sich in einem Übergangsbereich 62 zwischen dem dem distalen Endabschnitt 10 zugewandten Endbereich des ersten Absatzes 29 und einer ein distal endseitiges Lumen 61 begrenzenden Wandung eine Schulter 63 anschließt, die sich weiter nach radial innen erstreckt als der erste Absatz 29, sodass das Vorschubelement 40 in seiner Vorschubbewegung 53 durch diese Schulter 63 gestoppt wird.
Dadurch wird eine unbeabsichtigte Aufweitung des Katheterschaftes 20 außerhalb des mit dem Druckelement 30 versehenen Längenabschnitts unterbunden.
Ein (hier nicht dargestellter) Führungsdraht, der zur Positionierung des Katheterschaftes 20 durch diesen hindurch bis in das distal endseitige Lumen 61 führen kann, wird dadurch aber nicht behindert.

Mit dem erfindungsgemäßen Katheter 1 bzw. dem erfindungsgemäßen Katheter-System lassen sich somit in einfacher sowie zuverlässiger und kostengünstiger Weise Stenosen bzw. Ablagerungen in Gefäßen von Patienten durch Aufweitung und/oder Zerstörung vorbehandeln bzw. behandeln oder beseitigen. Bei Einsatz unterschiedlich dicker Vorschubelemente 40 können entsprechend unterschiedlich weite radiale Expansionen realisiert werden. Aufgrund der hohen Druckfestigkeit des Katheterschaftes 20 sowie des Druckelements 30 sind keine radialen Einschnürungen bzw. Verformungen zu befürchten. Die Länge der radialen Aufweitung lässt sich durch die Einschub-Länge des Vorschubelements 40 bzw. Dilatators beeinflussen und demzufolge auch die Länge der zu behandelnden Abschnitte des Gefäßes selbst.

## Patentansprüche

1. Katheter (1) zum Aufweiten und/ oder Zerstören einer Stenose in einem Gefäß eines Patienten, umfassend einen Katheterschaft (20) mit einem Hohlraum (24) und in dem Hohlraum (24) zumindest ein erstes, mit einer den Hohlraum (24) begrenzenden Wandung (25) fest verbundenes erstes Wirkelement (26) zur Ausbildung des Wirkprinzips der geneigten Ebene (50) zwecks Vergrößerung eines Abstandes (A) senkrecht zu einer Längsachse (21) des Katheterschaftes (20) und dadurch bedingter radialer Aufweitung des Katheterschaftes (20), **dadurch gekennzeichnet, dass** der Katheter (1) an der Außenseite des Katheterschaftes (20) angeordnet wenigstens ein radial vorstehendes Druckelement (30) aufweist zur Ausübung einer Druckkraft (31) auf eine Stenose in einem Gefäß.

2. Katheter nach Anspruch 1, **dadurch gekennzeichnet, dass** der Katheter des Weiteren ein Vorschubelement (40) zur Einführung und/ oder Bewegung in dem Hohlraum (24) entlang der Längsachse (21) des Katheterschaftes (20) umfasst, wobei das Vorschubelement (40) ein zweites Wirkelement (41) zur Ausbildung des Wirkprinzips der geneigten Ebene (50) aufweist, welches an dem ersten Wirkelement (26) anliegt oder anlegbar ist, so dass bei Relativbewegung zwischen den beiden Wirkelementen (26,41) eine Keilwirkung realisierbar ist, mit der eine radiale Aufweitung des Katheterschaftes (20) erzeugbar ist.

3. Katheter nach Anspruch 2, **dadurch gekennzeichnet, dass** eines der beiden Wirkelemente (26,41) eine Anlagefläche (27) und das jeweils andere Wirkelement (26,41) eine geneigte Fläche (43) aufweist, so dass bei Anlage der geneigten Fläche (43) an der Anlagefläche (27) und Ausführung einer Vorschubbewegung des Vorschubelements (40) das erste Wirkelement (26) nach radial außen bewegt wird.

4. Katheter nach Anspruch 3, **dadurch gekennzeichnet, dass** sich in axialer Richtung an die jeweilige Fläche (27,43) jeweils ein Absatz (29,52) anschließt, so dass nach keilwirkungsbedingter Aufweitung des Katheterschaftes (20) und unter Ausführung einer weiteren Vorschubbewegung die Absätze (29,52) radial aneinander zur Anlage bringbar sind.

5. Katheter nach einem der Ansprüche 2 bis 4, **dadurch gekennzeichnet, dass** das erste Wirkelement (26) sowie das zweite Wirkelement (41) in einer Ebene senkrecht zur Längsachse (21) vollständig um diese Längsachse (21) herum ausgebildet sind.

6. Katheter nach einem der Ansprüche 3 bis 5, **dadurch gekennzeichnet, dass** die geneigte Fläche (43) und / oder die Anlagefläche (27) in einer Ebene, in der die Längsachse (21) liegt, einen gewölbten Verlauf aufweist.

7. Katheter nach einem der Ansprüche 2 bis 6, **dadurch gekennzeichnet, dass** das Vorschubelement (40) eine biegbare Stange, insbesondere eine Schubstange, ist zur axialen Verlagerung des zweiten Wirkelements (41).

8. Katheter nach einem der vorhergehenden Ansprüche, **dadurch gekennzeichnet, dass** das Druckelement (30) eine längliche, entlang seiner Längserstreckung den Umfang des Katheterschaftes (20) umschließende Ausgestaltung aufweist.

9. Katheter nach Anspruch 8, **dadurch gekennzeichnet, dass** das Druckelement (30) helixförmig an der Außenseite (22) des Katheterschaftes (20) angeordnet ist.

10. Katheter nach Anspruch 9, **dadurch gekennzeichnet, dass** das Druckelement (30) in der Form einer Doppelhelix an der Außenseite (22) des Katheterschaftes (20) angeordnet ist.

11. Katheter nach einem der Ansprüche 9 und 10, **dadurch gekennzeichnet, dass** das Druckelement (30) an seinem radial äußeren Bereich eine geringere Breite aufweist als im Bereich seiner mechanischen Fixierung an der Außenseite (22) des Katheterschaftes (20).

12. Katheter nach einem der Ansprüche 10 und 11, **dadurch gekennzeichnet, dass** das Druckelement distal anschließend an die Helixform einen Ring umfasst, der in axialer Richtung/in Umfangsrichtung an der Außenseite des Katheterschaftes fixiert ist.

13. Katheter nach einem der vorhergehenden Ansprüche, **dadurch gekennzeichnet, dass** das Material des Katheterschaftes (20 ausgewählt ist aus Polypropylen, Polyethylen, Polyethylenterephthalat, Polyamid, Polyimid, Polyamid-Copolymere, Polyetherblockamid, Silikon oder silikon-basierten Materialien.

14. Katheter nach einem der vorhergehenden Ansprüche, **dadurch gekennzeichnet, dass** das Material des Druckelements (30) ausgewählt ist aus Nitinol, Edelstahl oder Polycarbonat.

15. Katheter-System, umfassend wenigstens einen Katheter (1) gemäß einem der Ansprüche 1-14 sowie mehrere Vorschubelemente (40) mit unterschiedlicher radialer Weite.
